# EUROPEAN PATENT SPECIFICATION

(11) **EP 0 292 399 B1**
(45) Date of publication and mention of the grant of the patent: **09.03.1994**
(21) Application number: 88401225.3
(22) Date of filing: 19.05.1988
(51) Int. Cl.: A61M 1/36

(54) **Blood reservoir for an extracorporeal blood treatment circuit**
Behälter für einen extrakorporalen Blutbehandlungskreislauf
Réservoir pour un circuit de traitement extra-corporel du sang

(30) Priority: 19.05.1987 JP 122243/87; 28.12.1987 JP 333608/87
(43) Date of publication of application: 23.11.1988
(73) Proprietor: TERUMO KABUSHIKI KAISHA, Tokyo 151 (JP)
(72) Inventor: Katsura, Yoshiro, Fuji-shi Shizuoka-ken (JP)
(74) Representative: Joly, Jean-Jacques

(56) References cited:
- FR-A- 2 452 936

## Description

The present invention relates to a blood reservoir for temporarily storing blood in an extracorporeal blood circulating circuit.

When a thoracic operation, for example, is to be carried out on a patient, an extracorporeal blood circulating circuit is established using an artificial lung in which the blood is circulated for an exchange of carbon dioxide and oxygen. The blood is controlled at a prescribed temperature by a heat exchanger, and thereafter the gases are exchanged in the artificial lung. Then, the blood is temporarily stored in a blood reservoir for a steady supply of the blood. The blood is pumped into the patient under the operation at a constant pulse rate.

Blood reservoirs for use in extracorporeal blood circulation include a closed-type blood reservoir in the form of a soft bag for storing blood in an airtight condition and an open-type blood reservoir in the form of a hard housing for storing blood. The open-type blood reservoir is advantageous in that priming and confirmation of the stored amount of blood can easily be performed, and it would be easy to construct the blood reservoir as a unitary component of an artificial lung. Therefore, various extracorporeal blood circulating apparatus employing open-type blood reservoirs have been proposed.

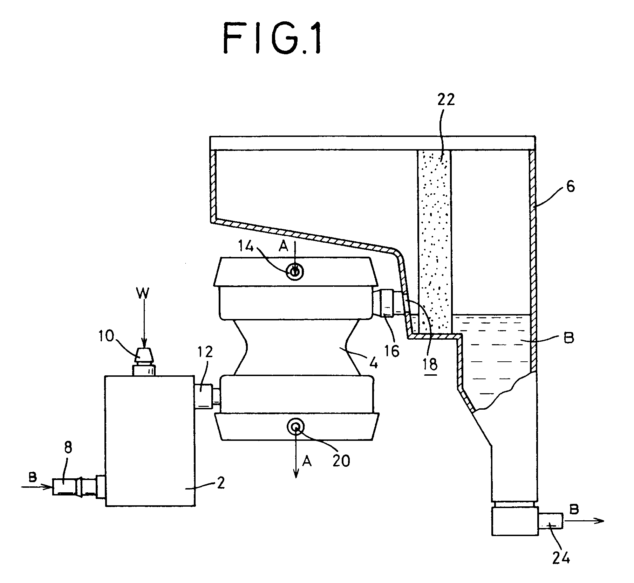

FIG. 1 of the accompanying drawings schematically illustrates an extracorporeal blood circulating apparatus proposed by the applicant. The extracorporeal blood circulating apparatus comprises a heat exchanger 2, an artificial lung 4, and a blood reservoir 6 as an interconnected unitary system. The extracorporeal blood circulating apparatus is connected to the body of a patient through a pump (not shown) which supplies blood at a certain pulse rate. Blood B discharged from the human body is introduced from a blood inlet port 8 into the heat exchanger 2 and controlled at a prescribed temperature by warm or cold water supplied from a water inlet port 10. Thereafter, the blood B is fed through a joint tube 12 into the artificial lung 4. The artificial lung 4 contains a multiplicity of hollow filamentary membranes through which a gas A containing oxygen supplied from a gas inlet port 14 flows. The blood B, while passing around the hollow filamentary membranes, receives oxygen from the gas A and discharges carbon dioxide, and then flows via a joint tube 16 through a blood inlet port 18 into the blood reservoir 6. After the exchange of oxygen and carbon dioxide, the gas A is discharged from the artificial lung 4 through a gas outlet port 20. The blood B that has entered the blood reservoir 6 from the blood inlet port 18 goes through an anti-foaming member 22 and is temporarily stored in the blood reservoir 6. Then, the stored blood B is supplied from a blood outlet port 24 into the human body by the pump.

The blood reservoir 6 incorporated in the extracorporeal blood circulating apparatus serves to temporarily store the blood B in order to supply the same at a constant rate and also to add various fluids, in particular drugs such as a vasodilatator drug, an anticoagulant, and the like to the blood B. It is desirable that these fluids be mixed uniformly with the blood B. Another example of a prior art blood reservoir, which corresponds to the preamble of claim 1, can be found in patent document FR-A-2 452 936

### SUMMARY OF THE INVENTION

It is then an object of the present invention to provide a blood reservoir allowing fluids, such as drugs, to be efficiently mixed with blood and a foam to be effectively eliminated from blood stored in the blood reservoir.

According to the invention, there is provided a blood reservoir for temporarily storing blood in an extracorporeal blood circulating circuit, comprising an anti-foaming filter which divides an interior space of the blood reservoir into a blood inlet section into which blood can flow and a storage section for storing blood, and a fluid charging inlet disposed in the blood inlet section for charging a fluid into the blood reservoir, said blood inlet section comprising at least one blood inlet, and characterised in that the fluid charging inlet is positioned above and substantially opposite to said blood inlet.

When the blood reservoir comprises a plurality of blood inlet ports, the fluid charging inlet preferably comprises a plurality of fluid inlet ports disposed respectively above the blood inlet ports.

The above and other features and advantages of the present invention will become more apparent from the following description when taken in conjunction with the accompanying drawings in which a preferred embodiment of the present invention is shown by way of illustrative example.

### BRIEF DESCRIPTION OF THE DRAWINGS

FIG. 1 is a schematic elevational view, partly in cross section, of an extracorporeal blood circulating apparatus shown as a comparative example;
FIG. 2 is a schematic elevational view, partly in cross section, of an extracorporeal blood circulating apparatus employing a blood reservoir according to the present invention; and
FIG. 3 is a schematic perspective view of the extracorporeal blood circulating apparatus shown in FIG. 2.

### DETAILED DESCRIPTION OF THE PREFERRED EMBODIMENT

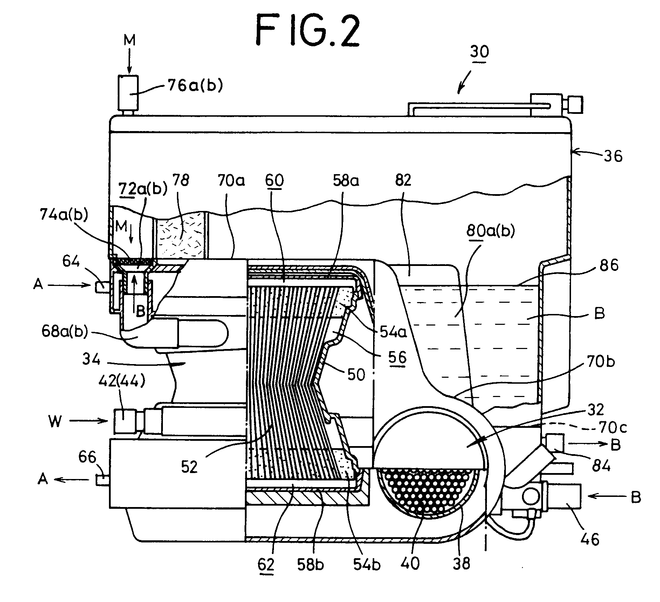

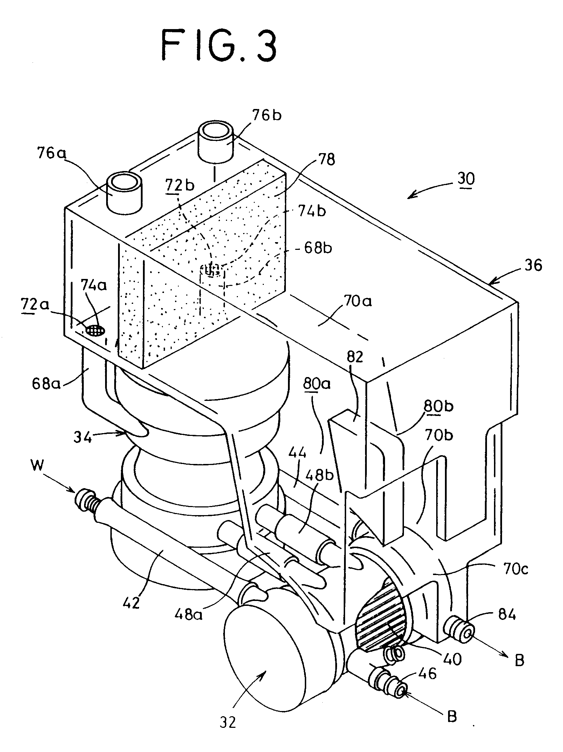

As shown in FIGS. 2 and 3, a blood reservoir according to the present invention is incorporated in an extracorporeal blood circulating apparatus which has a unitary apparatus assembly 30 comprising a heat exchanger 32, an artificial lung 34, and an open-type blood reservoir 36.

The heat exchanger 32 includes a tubular housing 38 accommodating therein a number of heat exchanger pipes 40. Warm or cold water W is supplied from a water port 42 to the pipes 40, and then discharged from the pipes 40 via a water port 44. Blood B flowing from a blood supply port 46 into the heat exchanger 32 is heated or cooled to a prescribed temperature while it is passing around the pipes 40. Thereafter, the blood B is supplied via two joint tubes 48a, 48b (see FIG. 3), to the artificial lung 34.

The artificial lung 34 serves to remove carbon dioxide from the blood B and add oxygen to the blood B. The artificial lung 34 includes a multiplicity of hollow filamentary membranes 52 bundled and stored in a substantially cylindrical housing 50 with a constricted central portion. On the upper and lower ends of the housing 50, there are mounted partitions 54a, 54b holding the opposite ends of the hollow filamentary membranes 52. The housing 50 and the partitions 54a, 54b jointly define a space surrounded thereby and housing the hollow filamentary membranes 52 therein, the space having a lower end communicating with the heat exchanger 32 through the joint tubes 48a, 48b. This space serves as a gas exchanging chamber 56 through which the blood B flows. Covers 58a, 58b are also mounted on the upper and lower ends of the housing 50. The partition 54a and the cover 58a define a gas supply chamber 60 therebetween, and the partition 54b and the cover 58b define a gas discharge chamber 62 therebetween. The hollow filamentary membranes 52 are supplied with a gas A containing oxygen from a gas inlet port 64 via the gas supply chamber 60. The gas A delivered from the hollow filamentary membranes 52 is discharged through the gas discharge chamber 62 from a gas outlet port 66.

The gas exchanging chamber 56 in the artificial lung 34 has an upper end communicating with the blood reservoir 36 through two L-shaped joint tubes 68a, 68b each having a horizontal portion extending from the gas exchanging chamber 56 and a vertical portion extending upwardly from the end of the horizontal portion over a certain length. The blood reservoir 36 has a bottom plate composed of first through third steps 70a, 70b, 70c. The joint tubes 68a, 68b have blood inlet ports 72a, 72b, respectively, opening at the first uppermost step 70a on its lateral sides through blood dispersing mesh screens 74a, 74b, respectively, for smoothing the blood flow flowing therethrough. The blood reservoir 36 is made of a transparent plastic material. The blood reservoir 36 has a pair of fluid inlet ports 76a, 76b above the blood inlet ports 72a, 72b, respectively, for adding various fluids, in particular drugs such as a vasodilatator drug, an anticoagulant, and the like to the blood B.

A urethane anti-foaming member 78 is disposed on the first step 70a of the blood reservoir 36 near the blood inlet ports 72a, 72b. A partition 82 is mounted substantially centrally on the second step 70b and divides the blood storage space on the second step 70b into a first blood storage region 80a and a second blood storage region 80b for preventing the blood surface from being subjected to resonant vibration. The third lowermost step 70c is coupled to a blood outlet port 84 for supplying the blood B stored in the blood reservoir 36 into the human body. In operation, the blood outlet port 84 is coupled to the human body through a pump (not shown) which delivers the blood at a prescribed pulse rate. In the case, the pump may be a rotary type or peristaltic finger type.

The extracorporeal blood circulating apparatus employing the blood reservoir of the present invention is basically of the above structure. Now, operation and advantages of the extracorporeal blood circulating apparatus will be described below.

The blood B coming from the blood supply port 46 is supplied through the gaps between the tubes 40 stored in the heat exchanger 32 to the artificial lung 34. At this time, water W maintained at a prescribed temperature is flowing through the heat exchanger tubes 40 through the water ports 42, 44. The blood B is heated or cooled to a predetermined temperature by the water W flowing through the tubes 40.

The blood B fed from the heat exchanger 32 via the joint tubes 48a, 48b into the artificial lung 34 enters the gas exchanging chamber 56 surrounded by the housing 50 in which oxygen is added to the blood B and carbon dioxide is removed from the blood B. More specifically, the gas A containing oxygen is supplied into the bundled hollow filamentary membranes 52 in the housing 50 from the gas inlet port 64 through the gas supply chamber 60. Oxygen and carbon dioxide are exchanged in the blood B through the hollow filamentary membranes 52. The gas A containing carbon dioxide is discharged via the gas discharge chamber 62 from the gas outlet port 66.

The blood B to which oxygen has been added in the artificial lung 34 then flows through the joint tubes 68a, 68b, the blood inlet ports 72a, 72b, and the mesh screens 74a, 74b into the blood reservoir 36. As described above, the fluid inlet ports 76a, 76b are disposed above the blood inlet ports 72a, 72b. A drug M such as a vasodilatator drug, an anticoagulant, or the like is charged through the inlet ports 76a, 76b into the blood reservoir 36 when the blood B flows into the reservoir 36. Since the drug M placed through the inlet ports 76a, 76b falls on the blood B as it flows out of the blood inlet ports 72a, 72b, the drug M can highly effectively be dispersed in the blood B. The blood reservoir 36 is divided by the urethane anti-foaming member 78 into a blood inlet section near the blood inlet ports 72a, 72b below the drug inlet ports 76a, 76b and the blood storage regions 80a, 80b for storing the blood B. The blood B mixed with the drug M is passed through the anti-foaming member 78 which promotes uniform mixture of the blood B and the drug M, and is then stored in the blood reservoir 36.

The blood B stored in the blood reservoir 36 is then intermittently or pulsatively delivered out to the human body by the pump (not shown) coupled to the blood outlet port 84. The surface level 86 of the blood B stored in the blood reservoir 36 would be subjected to resonant vibration due to intermittent delivery of the blood B by the pump. However, such resonant vibration is highly effectively suppressed by the partition 82 on the second step 70b in the blood reservoir 36. More specifically, the partition 82 located in the blood reservoir 36 is effective in making the system's natural frequency widely different from the pulse rate of the pump, thus suppressing the undesirable resonant vibration. Therefore, the surface level 86 of the blood B is kept calm without violent turbulent motion, and the height of the surface level 86, i.e., the amount of the blood B stored in the blood reservoir 36 can easily and accurately be checked by a visual inspection. Moreover, inasmuch as any turbulent motion of the blood B in the blood reservoir 36 is effectively suppressed, vibration of the blood B is held to a minimum, and no unwanted vibration is applied to the apparatus.

With the present invention, as described above, the blood reservoir for temporarily storing the blood has its interior space divided by the anti-foaming member into a blood inlet section into which the blood flows and a blood storage section for storing the blood, and has fluid inlet ports in the blood inlet section. A drug charged into the blood reservoir through the fluid inlet ports can effectively be dispersed in the blood by the flow of the blood into the blood reservoir. Since the fluid inlet ports are positioned in the blood inlet section, the added drug can be mixed with the blood more uniformly when the blood passes through the anti-foaming member.

## Claims

1. A blood reservoir (36) for temporarily storing blood in an extracorporeal blood circulating circuit, comprising an anti-foaming filter (78) which divides an interior space of the blood reservoir into a blood inlet section into which blood can flow and a reservoir section for storing blood, and a fluid charging inlet (76a, 76b) disposed in said blood inlet section for charging a fluid into the blood reservoir, said blood inlet section comprising at least one blood inlet (72a, 72b), characterized in that said fluid charging inlet (76a, 76b) is positioned above and substantially opposite to said blood inlet.

2. A blood reservoir according to claim 1, further characterized in that said blood inlet section comprises a plurality of blood inlet ports (72a, 72b), and in that said fluid charging inlet comprises a plurality of fluid inlet ports (76a, 76b) disposed respectively above and substantially opposite to a plurality of blood inlet ports.

3. An extracorporeal blood circulating apparatus comprising a heat exchanger (32), an artificial lung (34) and the blood reservoir of claim 1 or 2 interconnected together to provide a blood circulating circuit wherein blood enters first into said heat exchanger and then flows through said artificial lung upwardly into said blood reservoir.

## Patentansprüche

1. Blutbehälter (36) zum vorübergehenden Speichern von Blut in einem extrakorporalen Blutzirkulationskreislauf, der aufweist: einen Antischaum-Filter (78), der einen Innenraum des Blutbehälters in einen Bluteinlaßabschnitt, in den das Blut strömen kann, und einen Behälterabschnitt zum Speichern des Blutes teilt, und einen Fluideinfülleinlaß (76a, 76b), der zum Einfüllen eines Fluids in den Blutbehälter in dem Bluteinlaßabschnitt angeordnet ist, wobei der Bluteinlaßabschnitt mindestens einen Bluteinlaß (72a, 72b) aufweist und **dadurch gekennzeichnet ist, daß** der Fluideinfülleinlaß (76a, 76b) oberhalb und im wesentlichen gegenüber dem Bluteinlaß positioniert ist.

2. Blutbehälter gemäß Anspruch 1, desweiteren **dadurch gekennzeichnet,** daß der Bluteinlaßabschnitt eine Vielzahl von Bluteinlaßöffnungen (72a, 72b) aufweist, und daß der Fluideinfülleinlaß eine Vielzahl von Fuideinlaßöffnungen (76a, 76b) aufweist, die jeweils oberhalb und im wesentlichen gegenüber einer Vielzahl von Bluteinlaßöffnungen angeordnet sind.

3. Extrakorporales Blutzirkulationsgerät, das einen Wärmetauscher (32), eine künstliche Lunge (34) und den Blutbehälter nach Anspruch 1 oder 2 miteinander verbunden aufweist, um einen Blutzirkulationskreislauf zu schaffen, in welchem Blut zuerst in den Wärmetauscher eintritt und dann durch die künstliche Lunge nach oben in den Blutbehälter strömt.

## Revendications

1. Réservoir de sang (36) destiné au stockage temporaire de sang dans un circuit de circulation sanguine extracorporelle, comprenant un filtre antimousse (78) qui divise un espace interne du réservoir de sang en une partie d'admission de sang dans laquelle le sang peut s'écouler et une partie réservoir destinée au stockage du sang, et une admission destinée au chargement de fluides (76a, 76b) disposée dans ladite partie d'admission de sang, destinée au chargement d'un fluide dans le réservoir de sang, ladite partie d'admission de sang comprenant au moins une admission de sang (72a, 72b), caractérisé en ce que ladite admission servant au chargement de fluides (76a, 76b) est positionnée au-dessus et sensiblement à l'opposé de ladite admission de sang.

2. Réservoir de sang selon la revendication 1, caractérisé en outre en ce que ladite partie d'admission de sang comprend une pluralité d'orifices d'admission de sang (72a, 72b) et en ce que ladite admission servant au chargement de fluides comprend une pluralité d'orifices d'admission de fluides (76a, 76b) disposés respectivement au-dessus et sensiblement à l'opposé d'une pluralité d'orifices d'admission de sang.

3. Appareil de circulation sanguine extracorporelle comprenant un échangeur de chaleur (32), un poumon artificiel (34) et le réservoir de sang selon la revendication 1 ou 2 interconnectés pour fournir un circuit de circulation sanguine dans lequel du sang entre tout d'abord dans ledit échangeur de chaleur, puis est acheminé à travers ledit poumon artificiel vers le haut dans ledit réservoir de sang.
